# EUROPEAN PATENT APPLICATION

(11) **EP 1 488 813 A1**
(43) Date of publication of application: **22.12.2004**
(21) Application number: 02707127.3
(22) Date of filing: 22.03.2002
(51) Int. Cl.: A61K 48/00, A61K 47/42

(54) **COMPOSITIONS FOR DELIVERING BIOLOGICALLY ACTIVE DRUG AND METHOD OF USING THE SAME**

(71) Applicant: Anges MG, Inc., Toyonaka-shi, Osaka 560-0082 (JP)
(72) Inventor: Morishita, Ryuuichi, c/o Osaka University, Suita-shi, Osaka 565-0871 (JP); Taniyama, Yoshiaki, c/o Osaka University,, Suita-shi, Osaka 565-0871 (JP)
(74) Representative: Westendorp, Michael, Dr.
(86) International application number: PCT/JP2002/002755
(87) International publication number: WO 2003/080123

(57) **Abstract**

A composition for delivering a biologically active agent to a specific site in the living body comprises microbubble-encapsulated albumin microspheres containing perfluorocarbon. The biologically active agent is preferably at least one agent selected from the group consisting of oligonucleotides, antisense oligonucleotides, triple helix-forming oligonucleotides, oligonucleotide probes, nucleotide vectors, viral vectors, and plasmids. When the composition is used, the specific site is exposed to ultrasound so that the biologically active agent can be incorporated in the specific site.

## Description

### Technical Field

The present invention relates to a composition having a biologically active agent incorporated therein, characterized in that the composition comprises microbubble-encapsulated albumin microspheres containing perfluorocarbon, and to a method of using the same.

### Background Art

Ultrasonic diagnosis is a diagnostic method utilizing characteristics of ultrasound for noninvasively identifying, e.g., the distance between tissues or properties of a substance in the living body as real-time visual information. Ultrasonic diagnosis is advantageous in that a manipulation method is simple and a device is inexpensive, for example, and plays important roles in diagnosis, treatment, and the like of diseases in circulatory organs including the cardiac area, diseases in digestive organs, and malignant tumors. About fifty thousands of devices for ultrasonic diagnosis, which amount to several to several dozen times of MRI or CT devices, have been already domestically set up, whereby the diagnosis can be carried out in every region. Additional use of a contrast medium in such ultrasonic diagnosis provides a distinctive image, whereby the diagnosis can be carried out more accurately. Due to use of a contrast medium, ultrasonic diagnosis is expected to become more useful in diagnostics in the future.

Ultrasound is also used for enhancing absorption of various low-molecular-weight drug. For example, U.S. Patent No. 4953565 and U.S. Patent No. 5007438 disclose that percutaneous absorption of a drug is enhanced by ultrasonic vibration.

As an attempt of introducing a physiologically active substance into cells by using ultrasound, U.S. Patent No. 5315998 discloses that a composition containing microbubbles is combined with ultrasound to cause a drug to diffuse and penetrate. In addition, Tachibana et al. (Application of Ultrasound in the Drug Delivery System, Nippon Yakurigaku Zasshi, 114: 138-141, 1999) show that the effect of a drug in the living body is enhanced when ultrasound is used.

On the other hand, attempts of introducing a nucleic acid pharmaceutical agent such as a gene using ultrasound have been made. Tabata et al. (Application of Sonotechnology: Enhancement of the Effect of a Drug by Ultrasound, Chemical Engineering, 45: 254-257, 2000) show that a gene can be introduced into cultured cells by using ultrasound. WO 20000406 and WO 9933500 disclose that gene expression by plasmid DNA in the living body is enhanced by using ultrasound.

National Publication of International Patent Application No. 2000-507931 discloses that the amount of antisense nucleic acid incorporated in the living body by ultrasound increases, and gene expression by plasmid DNA is enhanced in a cultured cell line, by coexistence with a contrast medium used for ultrasonic diagnosis.

### Disclosure of the Invention

In view of such a situation, an object of the present invention is to provide a composition having a biologically active agent incorporated therein that can introduce a gene or the like directly into the living body and a method of using the same.

As a result of extensive studies to achieve the above object, the present inventors have found that a gene can be introduced into a specific site in the living body by ultrasonic irradiation by using a composition which comprises microbubble-encapsulated albumin microspheres filled with perfluorocarbon, the composition being essentially used as a contrast medium for ultrasonic diagnosis. This finding has led to the completion of the present invention.

Namely, the present invention provides a composition having a biologically active agent incorporated therein, characterized in that the composition is a composition for delivering a biologically active agent to a specific site in the living body, which comprises microbubble-encapsulated albumin microspheres containing perfluorocarbon.

The present invention also provides the composition having a biologically active agent incorporated therein, characterized in that the biologically active agent is at least one agent selected from the group consisting of oligonucleotides, antisense oligonucleotides, triple helix-forming oligonucleotides, oligonucleotide probes, nucleotide vectors, viral vectors, and plasmids.

The present invention further provides a method of using the composition having a biologically active agent incorporated therein, characterized in that the specific site is exposed to ultrasound so that the biologically active agent can be incorporated in the specific site.

### Brief Description of the Drawings

Figure 1 is a graph showing the number of survival days after administering a suicide gene;
Figure 2 is a graph showing the effect of the concentration of OPTISON on gene expression in the anterior cervical muscle;
Figure 3 is a graph showing the effect of the time of ultrasonic irradiation on gene expression in the anterior cervical muscle;
Figure 4 is a graph showing introduction of a gene into the vascular smooth muscle after a vascular disorder by a balloon catheter;
Figure 5 is a graph showing introduction of a gene into the vascular endothelial cells without a vascular disorder by a balloon catheter; and
Figure 6 is a graph showing the effect of gene introduction on the intima/media ratio in the artery after a vascular disorder by a balloon catheter.

### Best Mode for Carrying Out the Invention

The present invention will be described in detail below.

The composition having a biologically active agent incorporated therein according to the present invention is a composition for introducing a gene or the like directly into the living body and comprises, as a carrier, microbubble-encapsulated albumin microspheres containing perfluorocarbon that is an insoluble gas. This microbubble-encapsulated albumin microspheres containing perfluorocarbon is essentially used as a contrast medium for ultrasonic diagnosis.

There are no specific limitations to the microbubble-encapsulated albumin microspheres containing perfluorocarbon that is an insoluble gas, which are contained in the composition having a biologically active agent incorporated therein according to the present invention. However, the inventors have found, as a result of extensive studies, that OPTISON (trade name; manufactured by Molecular Biosystems, Inc., United States) is particularly preferably used as such microspheres. OPTISON has been already used as a contrast medium for ultrasonic diagnosis, and its safety is ensured.

There are no specific limitations to the biologically active agent that can be included by the composition having a biologically active agent incorporated therein according to the present invention. However, the composition preferably includes at least one biologically active agent selected from the group consisting of oligonucleotides, antisense oligonucleotides, triple helix-forming oligonucleotides (TFO), oligonucleotide probes, nucleotide vectors, viral vectors, and plasmids. Among them, a plasmid DNA which encodes a physiologically active gene is used particularly preferably.

A gene encoded by the plasmid DNA is not limited by a specific physiological activity. For example, a gene dealing with a disease, specifically, a gene counteracting a disease or a gene supplementing a disease-related deficiency is used. Examples of such a gene include genes encoding a pharmacologically active compounds selected from the group consisting of cytokines, growth factors, antibodies or antibody fragments, receptors for cytokines or growth factors, proteins having a proliferation-preventing or proliferation-inhibiting action, enzymes, pulse-related inhibitors, thrombus-inducing substances, and aggregation inhibitors; proteins having a fibrinolytic action, viral coat proteins, bacterial antigens, and parasitic antigens having a fibrinolytic action, tumor antigens, proteins having an effect on blood circulation , peptide hormones, and ribonucleic acids such as a ribozyme and antisense RNA.

The composition having a biologically active agent incorporated therein according to the present invention which comprises microbubble-encapsulated albumin microspheres containing perfluorocarbon, for example, can be obtained by mixing the above-described OPTISON as a carrier with the plasmid DNA as an agent. Namely, the microbubble-encapsulated albumin microspheres containing perfluorocarbon are associated with or located tightly close to the plasmid DNA as a biologically active agent by mixing these.

When the composition of the present invention reaches a specific site into which a gene encoded by the plasmid DNA included by the composition having a biologically active agent incorporated therein according to the present invention should be introduced, the specific site is exposed to ultrasound. The microbubbles within the microspheres burst due to ultrasonic energy to give an impact on the cells present at the specific site to such an extent that it does not affect the safety, whereby the biologically active agent such as a gene can be introduced into the cells.

Accordingly, the present invention includes a method of using the composition having a biologically active agent incorporated therein, comprising a step of administering the composition having a biologically active agent incorporated therein according to the present invention and a step of exposing a recognition site (the above-described specific site) to ultrasound so that the biologically active agent can be incorporated in the specific site in the living body.

There are no specific limitations to the specific site into which a gene can be introduced by such a method. Examples of the specific site include brain, digestive organs, blood vessels, muscles, and various disease sites. The gene is administered particularly preferably to a tumor tissue.

### (Examples)

The present invention will be described in more detail by Examples, but the present invention is not limited by these Examples.

### [Example 1]

### Experiment of introducing a gene into brain

A male WKY rat weighing 350 to 400 g was anesthetized with Nembutal (0.1 ml/100 g). Then, the vertex was shaved with a clipper, and a PE10 tube was cannulated into the left external carotid artery. Then, a probe for ultrasonic transmission was fixed on the vertex with a gel interposed therebetween. 1 ml of a solution of a plasmid (pLuc) encoding a luciferase gene, a mixed solution of pLuc and OPTISON, or a mixed solution of pLuc and LEVOVIST (trade name; manufactured by Molecular Biosystems, Inc.) as a contrast medium for ultrasonic diagnosis was arterially injected into the left internal carotid artery slowly in two minutes, and, at the same time, the skull was continuously irradiated with 2.5 W/cm² or 5 W/cm² of ultrasound for two minutes.

After 24 hours, the brain (cerebrum, mesencephalon, cerebellum, medulla oblongata) was taken out and divided into the left brain and the right brain to determine the activity of luciferase. In the injection, OPTISON was used as an undiluted drug solution, and LEVOVIST was used as a solution having a concentration of 300 mg/ml.

Consequently, the efficiency of pLuc introduction was most excellent when a mixture of pLuc and 0.1 ml of OPTISON was administered and 5 W/cm² of ultrasound was irradiated. Luciferase expression was not observed when only pLuc was administered and no ultrasonic was irradiated, when the mixed solution of pLuc and OPTISON was administered and no ultrasonic was irradiated, or when only pLuc was administered and ultrasonic was irradiated. Further, gene expression was higher in the case of using OPTISON than in the case of using LEVOVIST as a carrier (contrast medium for ultrasonic diagnosis) in the composition of the present invention. Gene expression was confirmed only in the left brain into which pLuc was arterially injected. On the other hand, it was confirmed that the rat had a burned scalp since the surface temperature of the skull was raised to up to 60°C due to the ultrasonic irradiation. It is believed that such a burn can be prevented by changing from a high-frequency ultrasound at 1 MHz used now to a low-frequency ultrasound at about 40 to 200 KHz that can easily be transmitted into the skull, and correspondingly reducing the ultrasonic intensity.

### [Example 2]

### Experiment of introducing a gene into tumor tissue

5 × 10⁶ B16-F1 cells were inoculated into the back of a male C57BL/6J mouse. One week later, 50 µl of a solution (1 µg/µl) of a plasmid (pTK) which encoded a herpes thymidine kinase gene that was a suicide gene or a mixed solution of pTK and 50%-concentrated OPTISON was topically injected into the tumor tissue. Irradiation with ultrasound with an intensity of 10 at 2 Hz for 30 seconds was then carried out four times. Six days after introducing the gene, 100 mg/kg of ganciclovir was administered for five consecutive days to determine the number of survival days.

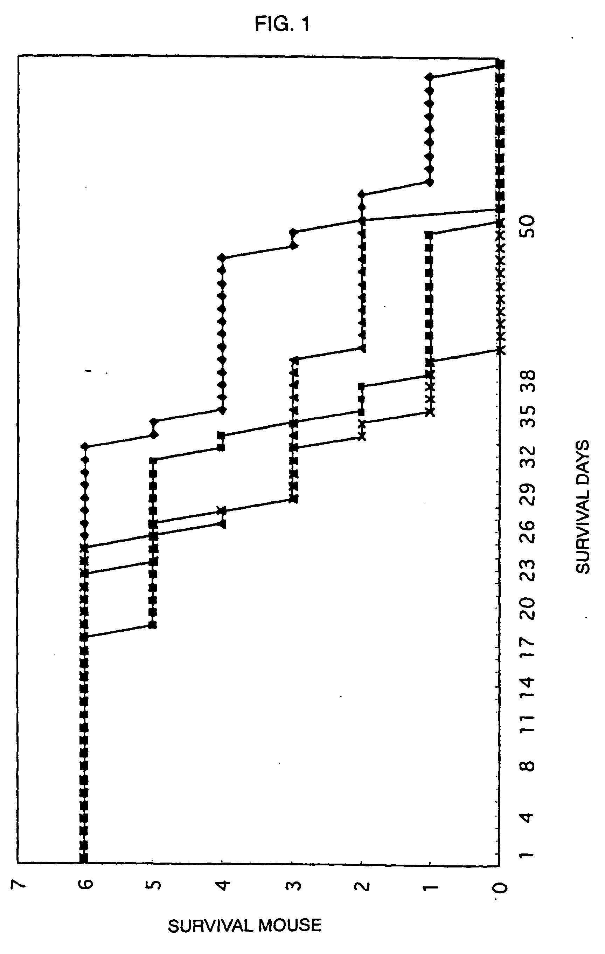

The results are shown in the graph of Figure 1. In Figure 1, the symbol ◆ denotes a series in which ultrasonic irradiation was carried out after administering the mixed solution of pTK and OPTISON; the symbol ■ denotes a series in which ultrasonic irradiation was carried out after administering the pTK solution; the symbol ▲ denotes a series in which ultrasonic irradiation was not carried out after administering the mixed solution of pTK and OPTISON; and the symbol × denotes a series in which ultrasonic irradiation was not carried out after administering the pTK solution.

Based on the results in Figure 1, prolongation of survival days was observed in the series in which ultrasonic irradiation was carried out after administering the mixed solution of pTK and OPTISON. It was made clear that the gene was efficiently introduced into the living body by ultrasonic irradiation in the presence of OPTISON that was a contrast medium for ultrasonic diagnosis.

### [Example 3]

### Experiment of introducing a gene into skeletal muscle

Amale WKY rat with aweight of 350 to 400 g was anesthetized with Nembutal (0.1 ml/100 g). Then, the neck was shaved with a clipper. Then, a probe for ultrasonic transmission was fixed on the neck with a gel being interposed therebetween. 50 µl of a mixed solution of 20 µg of pLuc and OPTISON was intramuscularly injected into the anterior cervical muscle, and, at the same time, the neck was irradiated with 2.5 W/cm² of ultrasound at 1 MHz. 24 hours later, the anterior cervical muscle was taken out to determine the activity of luciferase.

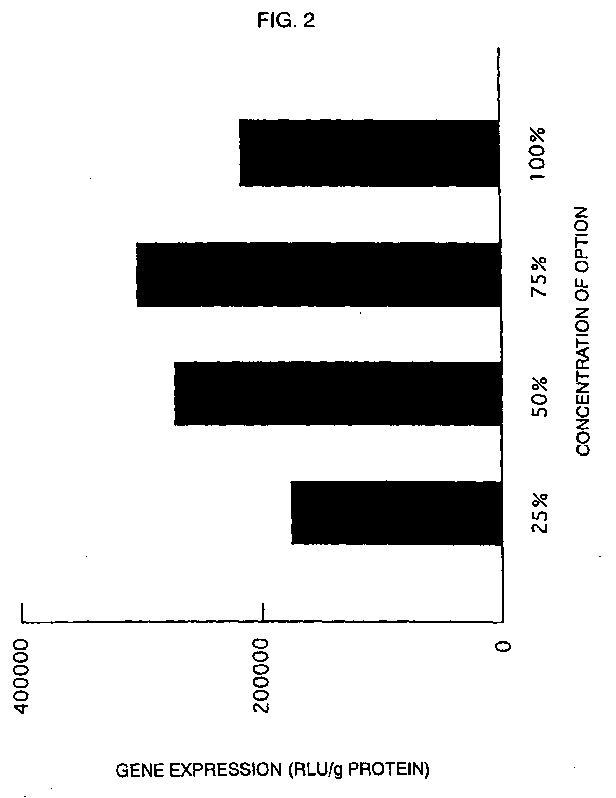

As a result of comparing the efficiency in introducing the gene by changing the concentration of OPTISON in the mixed solution administered, the highest gene expression was observed when the concentration of OPTISON was 75% (Figure 2).

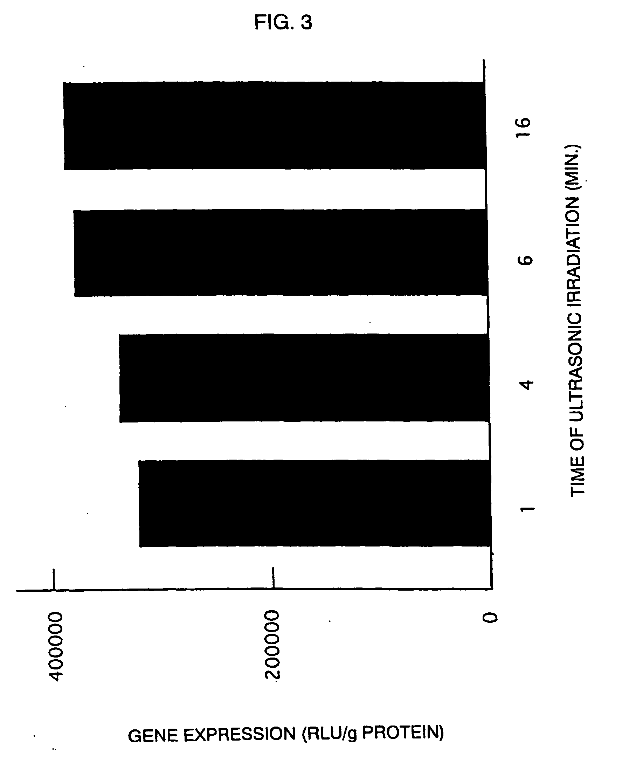

As a result of examining the effect of the time of ultrasonic irradiation when a mixed solution of pLuc and 75%-concentration OPTISON was administered, it was shown that the efficiency in introducing the gene was improved as the time of ultrasonic irradiation was longer, which showed that ultrasonic irradiation in the presence of OPTISON that was a contrast medium for ultrasonic diagnosis was advantageous (Figure 3).

### [Example 4]

### Experiment of introducing a gene into blood vessel

A male WKY rat with a weight of 350 to 400 g was anesthetized with Nembutal (0.1 ml/100 g). Then, the neck was shaved with a clipper. Then, a probe for ultrasonic transmission was fixed on the neck with a gel interposed therebetween. 50 µl of a solution of 50 µg of pLuc or a mixed solution of pLuc and 10%-concentrated OPTISON was arterially injected into the left external carotid artery of the rat of which the vascular endothelial cells were damaged by introducing a balloon catheter into the left external carotid artery or of which the vascular endothelial cells were not damaged, and, at the same time, the neck was irradiated with 2 W/cm² of ultrasound at 1 MHz in two minutes. 24 hours later, the left external carotid artery was taken out to determine the activity of luciferase.

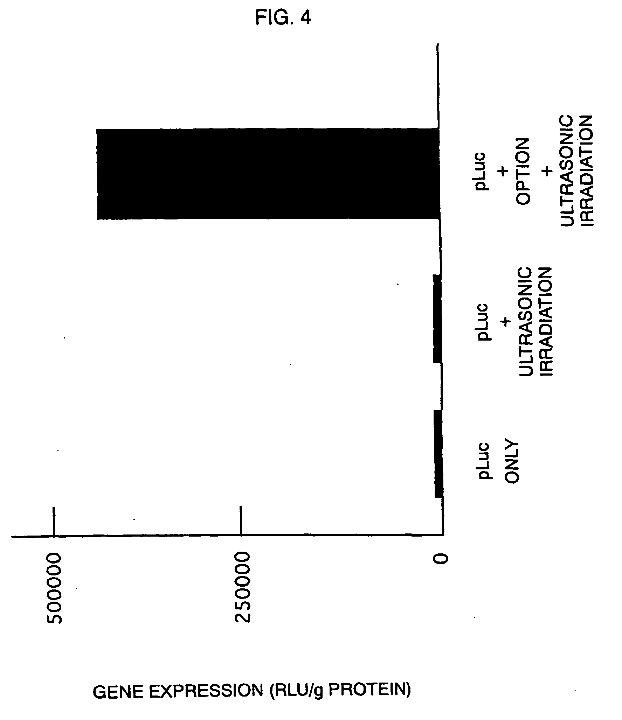

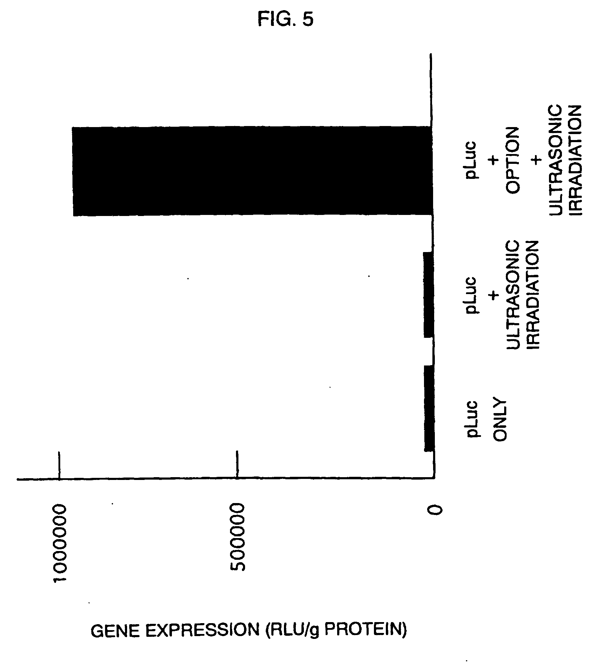

High gene introduction was observed only when the mixed solution of pLuc and OPTISON was administered to the rat of which the vascular endothelial cells were damaged by a balloon catheter (Figure 4) or of which the vascular endothelial cells were not damaged (Figure 5), which showed that ultrasonic irradiation in the presence of OPTISON was advantageous.

Further, 50 µl of a mixed solution of 100 µg of a plasmid (pP53) which encoded a tumor suppressor gene p53 which is believed to have an effect of inhibiting cell proliferation, and 25% OPTISON, was arterially injected into the left external carotid artery of the rat of which the vascular endothelial cells were damaged by a balloon catheter, and, at the same time, the neck was irradiated with 2 W/cm² of ultrasound at 1 MHz in two minutes. Two weeks later, the left external carotid artery was taken out to determine the intima/media ratio in the artery.

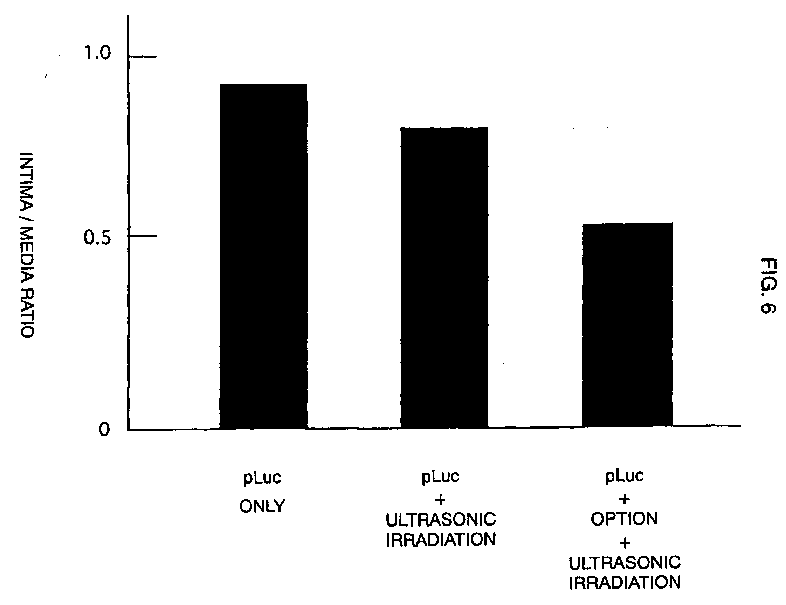

Consequently, the intima/media ratio in the artery was controlled at a low level when the mixed solution of pP53 and OPTISON was administered (Figure 6), which showed that the mixed solution was applicable to treatment of restenosis.

### Industrial Applicability

Since the composition having a biologically active agent incorporated therein according to the present invention uses a specific contrast medium for ultrasonic diagnosis as a carrier, a gene can be safely and securely introduced directly into the living body by ultrasonic irradiation.

## Claims

1. A composition having a biologically active agent incorporated therein, **characterized in that** the composition is for delivering the biologically active agent to a specific site in the living body, and comprises microbubble-encapsulated albumin microspheres containing perfluorocarbon.

2. The composition having a biologically active agent incorporated therein according to claim 1, **characterized in that** the biologically active agent is at least one agent selected from the group consisting of oligonucleotides, antisense oligonucleotides, triple helix-forming oligonucleotides, oligonucleotide probes, nucleotide vectors, viral vectors, and plasmids.

3. A method of using the composition having a biologically active agent incorporated therein according to claim 1 or 2, **characterized in that** the specific site is exposed to ultrasound so that the biologically active agent can be incorporated in the specific site.
